# NEW EUROPEAN PATENT SPECIFICATION

(11) **EP 0 597 852 B2**
(45) Date of publication and mention of the opposition decision: **10.08.2005**
(45) Mention of the grant of the patent: 03.12.1997
(21) Application number: 91911598.0
(22) Date of filing: 24.05.1991
(51) Int. Cl.: A61K 39/02

(54) **SWINE PNEUMONIA VACCINE AND METHOD FOR THE PREPARATION THEREOF**
IMPFSTOFF GEGEN DIE PNEUMONIE BEI SCHWEINEN UND VERFAHREN ZU SEINER HERSTELLUNG
VACCIN CONTRE LA PNEUMONIE PORCINE ET PROCEDE DE PREPARATION DUDIT VACCIN

(30) Priority: 29.05.1990 US 530669; 31.08.1990 US 575921; 26.12.1990 US 634237
(43) Date of publication of application: 25.05.1994
(73) Proprietor: Wyeth Holdings Corporation, Madison, NJ 07940 (US)
(72) Inventor: DAYALU, Krishnaswamy, I., Lincoln, NB 68506 (US); PEETZ, Richard, H., Lincoln, NB 68503 (US); FRANTZ, Joseph, C., Lincoln, NB 68516 (US); ROBERTS, David, S., Lincoln, NB 68506 (US); SWEARINGIN, Leroy, A., Lincoln, NB 68510 (US); KEMMY, Richard, J., Gretna, NB 68028 (US)
(74) Representative: VOSSIUS & PARTNER
(86) International application number: PCT/US1991/003689
(87) International publication number: WO 1991/018627

(56) References cited:
- EP-A- 0 367 757
- FR-A- 2 073 358
- AMERICAN JOURNAL OF VETERINARY RESEARCH vol. 45, no. 10, October 1984, pages 1899 - 1906 RICHARD F. ROSS ET AL. 'Characteristics of protective activity of Mycoplasma hyopneumoniae vaccine'
- A. Hyg. Camb., Volume 67, issued 1969, GOODWIN et al., "Some experiments relating to artificial immunity in enzootic pneumonia of pigs", pages 465-475, see summary.
- Acta Veterinaria (Beograd), Volume 25, No. 4, issued 1975, DURISIC et al., "Antibodies in blood, colostral and milk sera of sows inoculated with an experimental vaccine of Mycoplasma suipneumoniae", pages 189-194, see summary.
- Ross et al, J Vet Research, vol.35,no.10,p.1899-1906
- ANIMAL PHARM no.208, July 1990, p.20
- ANIMAL PHARM no.210, August 1990, p.21
- Extract from Rhone Merieux catalogue July 1984

## Description

### Field of the Invention

This invention relates to vaccines against infection by pathogens of the genus Mycoplasma and, more particularly, to bacterins useful as prophylactic agents to inhibit infection by Mycoplasma, particularly Mycoplasma hyopneumoniae.

### Background of the Invention

Mycoplasma hyopneumoniae is a ubiquitous swine respiratory pathogen causing mycoplasmal pneumonia in swine (MPS). MPS occurs worldwide and is considered to be one of the most common and economically important diseases affecting swine. Transmission of M. hyopneumoniae apparently occurs through direct contact with infected respiratory-tract secretions or aerosol droplets. It has been identified as the primary pathogen in the enzootic pneumonia complex. This disease has been estimated to cost in excess of $100 million annually, primarily due to its adverse effects on affected animals.

M. hyopneumoniae infection results in a chronic infection causing a lingering nonproductive cough and stunted growth, resulting in overt disease symptoms at the "growing and finishing stage", i.e., 6 weeks of age or older, in pigs. Severe lung damage to the infected animal, and/or death may occur at 4 to 6 months of age. Because most naturally occurring cases of MPS are mixed infections involving mycoplasmas, bacteria, viruses and parasites, death of the animal is usually due to secondary bacterial, viral or other pathogenic infections.

In the twenty-five years since the discovery of Mycoplasmas as the causative agent for enzootic pneumonia in 1965, researchers have pursued a variety of means to control and treat this disease and develop a safe and effective vaccine against Mycoplasma hyopneumoniae. Protection against infection induced by vaccine candidates is preferably measured by reduction in the number of lung lobes with lesions, reduction in the percent of lung lobes with lesions, reduction in mean lung lesion scores, reduction in microscopic lesion scores, and reduction in severity of M. hyopneumoniae infection as measured by a fluorescent antibody test.

Several experimental vaccines have produced less than optimal results. Studies have shown that strong immunity developed during the course of experimentally-induced MPS. However, when pneumonic lung tissue is administered as an antigen, animals become even more susceptible to challenge. Formalinized cultures of M. hyopneumoniae have been shown not to be protective. [See, also, e.g., C. A. Brandly et al, eds., "Advances in Veterinary Science and Comparative Medicine", Vol. 17, Academic Press, NY (1973) and references cited therein; R.F.W. Goodwin et al, A. Hyg. Camb., 67:465 (1969)]. Extracts of M. hyopneumoniae in ether or sodium dodecylsulfate (SDS), and a repeat freezethaw product have been shown to give significant protection against lung homogenate challenge [K.M. Lam et al, Am. J. Vet. Res., 32:1737 (1971)].

G. Christianson et al. (Chemical Abstracts, 1980, Abstract Number 199756) discloses that binary ethyleneimine was not consistently successful in inactivating mycoplasma contaminants in serums. FR-A-2.073.358 discloses multivalent vaccines and their methods of preparation. In particular, the prior art including FR-A-2.073.358 and Chemical Abstracts, 1980, Abstract Number 19975p does not disclose a vaccine component comprising M. hyopnemoniae inactivated with binary ethyleneimine at a dosage level of at least 5 x 10⁸ CCU, said component being capable of inducing an immunological response in vaccinated swine against M. hyopneumoniae.

Sonicated cells of M. hyopneumoniae in Tween 80 and paraffin oil resulted in good indirect hemagglutination (IHA) titers in the blood, colostrum and milk when injected into the mammary gland [S. Durisic et al, Acta Vet. (Beograd), 25(4):189-194 (1975)]. Whole cell preparations and cell-free supernatants have been shown to be only partially protective [R.F. Ross et al, Am. J. Vet. Res., 45(10):1899 (1984)]. Plasma membranes from sonically disrupted cells, adjuvanted with Al₂(OH)₃ or agarose, gave good passive protection [M. Kobisch et al, Ann. Inst. Pasteur/Immunol., 138:693-705 (1987)].

A live, avirulent LKR strain of M. hyopneumoniae was also shown to give good protection in pigs against challenge with a virulent M. hyopneumoniae strain [L. C. Lloyd et al, Abstract 4593 in Bacteriology and Bacterial Diseases, from Australian Vet. J. 66(1): 9-12 (1989)].

Infection by M. hyopneumoniae is presently controlled, in part, with various classes of antibiotics, such as tetracycline, lincomycin, add tiamulin. However, antibiotics are of limited therapeutic value because they do not prevent the establishment of an infection, and lung lesions may develop after treatment ends. The presence of secondary pathogens also makes selection of the appropriate antibiotic difficult [R. Landon, Topics in Vet. Med., 1(1):14-21 (1990)].

Other approaches which are presently in use to reduce the impact of chronic respiratory diseases caused by M. hyopneumoniae, are minimal disease systems such as the Swedish and British systems and farrowing of older sows. Minimization of stress, optimal management conditions and all-in, all-out systems of production are recommended.

There remains a need in the art for an effective vaccine against M. hyopneumoniae, which would confer protection against M. hyopneumoniae challenge and also significantly reduce the morbidity and mortality from secondary respiratory pathogens, such as Pasteurella multocida.

### Summary of the Invention

In one aspect the invention relates to a composition useful for vaccinating pigs against Mycoplasma hyopneumoniae. This composition comprises inactivated M. hyopneumoniae organisms in a pharmaceutically acceptable medium, the organisms being present in an amount sufficient to induce an immunological response in pigs protective against challenge by M. hyopneumoniae.

In a further aspect, the present invention provides a vaccine composition comprising the inactivated M. hyopneumoniae composition above in combination with additional vaccine components, including one or more components capable of inducing protection against athrophic rhinitis following infection by P. multocida, and other agents infectious for swine. Such vaccines may include immunogenic amounts of one or more of the following vaccine components: a stable, soluble, cell-free toxoid of P. multocida, a whole Pasteurella multocida bacterin with cell-bound toxoid, a B. bronchiseptica bacterin or an Erysipelothrix rhusiopathiae bacterin-extract, an Actinobacillus pleuropneumoniae bacterin, a Haemophilus parasuis bacterin, and/or viral vaccine components, such as from a Pseudorabies virus. Other conventional vaccine components may also be added to the vaccine compositions of this invention.

As still another aspect, the invention provides a method for producing the vaccine components described above. The M. hyopneumoniae vaccine component is prepared by a series of steps including pretreatment of the culture medium with ion exchange resins, such as the Amber-lites resin, increasing the dissolved oxygen content of the inoculated culture to between 20 and 40%, and inactivating the culture with a selected inactivating agent.

Yet a further aspect of this invention includes a method for increasing the resistance of swine to M. hyopneumoniae infection comprising administering an effective amount of the vaccine compositions of the present invention to swine.

Still another aspect of this invention includes a method for protecting swine against M. hyopneumoniae infection and other pathogenic infections comprising sequentially or simultaneously administering to an animal an effective amount of the M. hyopneumoniae vaccine and one or more vaccines containing an additional antigen, e.g., from pathogens as identified above.

Other aspects and advantages of the present invention are described in the following detailed description of preferred embodiments of the present invention.

### DETAILED DESCRIPTION OF THE INVENTION

The present invention provides vaccine components, vaccine formulations, and methods for their preparation and use in pigs as an aid in the prevention of infection by M. hyopneumoniae. The vaccine components and vaccines of this invention confer protection against M. hyopneumoniae challenge with a wild-type strain as well as other known virulent strains. Embodiments of the invention are also capable of significantly reducing the morbidity and mortality from secondary respiratory pathogens, such as Pasteurella multocida.

Mycoplasma hyopneumoniae strains useful in preparing the vaccines of the present invention can be isolated from swine infected with wild-type or other known strains causing mycoplasmal pneumonia in swine. Other known strains of M. hyopneumoniae, both virulent and non-virulent, may be useful in the compositions of this invention. Useful strains may be obtained from commercial or academic collections, such as the American Type Culture Collection in Rockville, Maryland, U.S.A.

A vaccine component according to this invention may be prepared by inoculating a medium capable of supporting the growth of Mycoplasma, particularly M. hyopneumoniae, with a selected M. hyopneumoniae seed stock. A selected culture medium may include media known to those of skill in the art to propagate Mycoplasma, such as described in Freundt, cited above, and other prior art. A particularly desirable medium formulation for culturing of the M. hyopneumoniae is described in Example 1 below and includes PPLO broth, yeast extract, heat inactivated serum, cysteine hydrochloride, dextrose, an antibiotic to inhibit bacterial growth, and an optional agent to indicate growth and to avoid excess pH change, e.g., phenol red.

Preferably, the selected medium is pretreated with an anion exchange resin, Amberlite (chloride form) resin. This step is believed to enhance growth of the organism by removing inhibitory agents. Other conventional methods, such as gel filtration, may also prove useful in this pretreatment step. This pretreatment step is preferably applied to the broth before addition of the other medium components and prior to inoculation. Exposure to the ion exchange resin may be maintained between one to four hours at a rate of approximately 500 grams/10 liters of broth:

Once pretreated, the medium is inoculated with a suspension of the selected M. hyopneumoniae strain, at preferably a 5-20% inoculation. The culture is thereafter incubated at a temperature of between approximately 30°C to 40°C. A more preferred temperature range is 35°C to 38°C, with 37°C being particularly preferred.

In a particularly preferred embodiment, the dissolved oxygen content of the culture is raised to a level of between 20% to 40% of saturation. A preferred oxygen content of the culture during the incubation is approximately 25%. Cultures containing a dissolved O₂ content of greater than 20%, produce higher titers of the organism. Increasing the dissolved oxygen content is accomplished by conventional means in the art, such as by aeration with sterile air and agitation. This particular step in the culturing of the vaccine component is believed to provide enhanced titers of M. hyopneumoniae.

The pH of the culture is maintained at neutral to slightly alkaline pH. Desirably the pH of the culture is maintained between 6.2 to 7.9. A more preferable pH range is 7.0 ± 0.5. The desired pH may be maintained by the addition of sterile NaOH, when necessary.

Cultures are incubated for 36 to 168 hours. More preferably the incubation period is between 36 and 96 hours, until a minimum titer of 1 X 10⁸ color changing units (CCU) in liquid titration medium [A.W. Rodwell and R.H. Whitcomb (1983) in Methods in Mycoplasmology, Vol. 1, Chapter 14; Shmuel Razin and Joseph G. Tulley, Eds.] is achieved. More preferably the titer is at least 5 X 10⁸ CCU/ml, and may be up to 5 X 10¹⁰ CCU/ml as determined by CCU or an ELISA.

At the end of the culture period, the organisms are inactivated by the addition of a known inactivating agent. One such preferred inactivating agent is binary ethyleneimine (BEI). Other inactivating agents may include, for example, formaldehyde or glutaraldehyde. The inactivating agent is added in an approximate amount of 4.0 mM. Once the inactivating agent is added, the culture is incubated again with agitation for at least 24 hours.

The inactivating agent may be removed or neutralized by conventional means, and the culture incubated again for at least 24 hours to complete neutralization of the inactivating agent. Once inactivated, the vaccine component may be formulated into a vaccine for administration to animals.

The present invention also contemplates vaccine components for use in combination with the M. hyopneumoniae vaccine component described above. Vaccine components, including inactivated bacterins or purified toxoids, from one or more pathogens, such as Pasteurella multocida, Streptococcum suis, Actinobacillus pleuropneumoniae, Haemophilus parasuis, Bordetella bronchiseptica, Salmonella choleraesuis and ascaris larva, may also be employed in conjunction with the vaccine components described above in combination vaccines or in therapeutic methods involving sequential or simultaneous co-administration. Such a combination vaccine is prepared by mixing an immunogenic amount of inactivated M. hyopneumoniae as described above and an immunogenic amount of a bacterin or toxoid of another pathogen with suitable adjuvants and physiologic vehicles for injection into mammals. A co-administration therapy may employ one or more of the above antigens formulated into individual vaccines. The M. hyopneumoniae vaccine and additional selected vaccine may be administered via the same routes of administration, using different sites for administration. Administration may be sequential or simultaneous.

Preferred embodiments of such combination vaccines or vaccine co-administration therapies include immunogenic amounts of one or more of the following vaccine components: a stable, soluble, cell-free toxoid of P. multocida, a whole Pasteurella multocida bacterin with cell-bound toxoid, whole cell P.multocida, type A and type D, a B. bronchiseptica bacterin or an Erysipelothrix rhusiopathiae bacterin-extract.

Additional vaccine combinations with the M. hyopneumoniae component of this invention may include the antigens Actinobacillus pleuropneumoniae, Haemophilus parasuis, and Pseudorabies virus. These additional components may be useful in a vaccine formulation or therapy for weaned pigs, preferably for administration as early as 3 to 6 weeks of age.

Based on at least one challenge, it appears that a vaccine co-administration with one vaccine containing the M. hyopneumoniae vaccine component and a second vaccine containing a P. multocida vaccine component referred to above evidenced no immunosuppressive effects by the M. hyopneumoniae vaccine on the animals as determined by seroconversion responses.

Vaccines of the invention may be prepared as pharmaceutical compositions containing an effective immunogenic amount of the inactivated organism, as active ingredients in a nontoxic and sterile pharmaceutically acceptable carrier. Such a vaccine may comprise the inactivated vaccine component described above mixed with optional preservatives and emulsifiers.

Alternatively or additionally, the inactivated M. hyopneumoniae may be admixed or adsorbed with a conventional adjuvant. The adjuvant is used as a non-specific irritant to attract leukocytes or enhance an immune response. Such adjuvants include, among others, Amphigen, mineral oil and lecithin, aluminum hydroxide, muramyl dipeptide, and saponins such as Quil A.

A preferred embodiment of the vaccine of the invention contains an aqueous suspension or solution containing the inactivated P-5722-3 strain of M. hyopneumoniae, preferably buffered at physiological pH, in a form ready for injection.

It is preferred that the vaccine of the invention, when in a pharmaceutical preparation, be present in unit dosage forms. For purposes of this invention, a desirable immunogenic amount of inactivated organism, when administered as the sole active ingredient is between 5 X 10⁸ (CCU) and 5 X 10⁹ (CCU). The vaccine of the invention is preferably administered in two 2 ml doses, each dose containing the desired titer. Preferably the doses are administered two weeks apart.

Primary immunization of piglets should be initiated at approximately one week of age with a booster dose 2 weeks later. For primary immunization of pregnant swine, two doses are recommended approximately four weeks apart with the last dose administered two weeks before farrowing. A booster dose is recommended prior to each subsequent farrowing. Semi-annual vaccination is recommended for boars.

It is preferred that the vaccine of the invention, when in a pharmaceutical preparation, be present in unit dosage forms. Dosage forms preferably contain about 2 ml. For purposes of this invention, an immunogenic amount of the inactivated M. hyopneumoniae, when administered as the sole active ingredient is between about 5X10⁸ to 5X10⁹ CCU per dose. In a vaccine composition containing additional antigenic components, the same immunogenic amount or a reduced amount of M. hyopneumoniae may be employed.

Other appropriate therapeutically effective doses can be determined readily by those of skill in the art based on the above immunogenic amounts, the condition being treated and the physiological characteristics of the animal. In the presence of additional active agents, these unit dosages can be readily adjusted by those of skill in the art. A desirable dosage regimen involves administration of one or two doses of desired vaccine composition, where the antigenic content of each fraction is desirably as stated above. Of course, the administration can be repeated at suitable intervals if necessary or desirable.

The mode of administration of the vaccines of the invention may be any suitable route which delivers the vaccine to the host. However, the vaccine is preferably administered by intramuscular injection. Other modes of administration may also be employed, where desired, such as subcutaneously, intradermally, intraperitoneally or intranasally.

The following Examples of the invention are illustrative only and not intended to be limiting.

### EXAMPLE 1

### Propagation and Culture of M. hyopneumoniae

M. hyopneumoniae strain P-5722-3 was furnished courtesy of Dr. Charles Armstrong, Purdue University, and deposited with the American Type Culture Collection under Accession No. 55052. This strain has the immunochemical and biochemical characteristics of being mannose positive, arginine negative, and urease negative. The strain is positive for growth inhibition with anti-M. hyopneumoniae antiserum and positive by direct fluorescent antibody test with anti-hyopneumoniae fluorescein-conjugated antibody. This strain was propagated as described below.

A culture medium was prepared according to the following procedure. An 83% PPLO broth, without crystal violet [Difco Laboratories, Detroit, Michigan] was conditioned by treating the broth with an anion exchange resin (Amberlite®, Sigma IRA400-chloride form] for one to four hours, at the rate of 500 grams of resin for every ten liters of broth.

Yeast extract was prepared by adding five hundred grams of active yeast granules to three liters of distilled or deionized water, stirred at room temperature. After thorough mixing, the suspension was stirred for an additional 15-45 minutes after which 16.2 ml of 10 N NaOH was added, dropwise. The slurry was then autoclaved for 15-45 minutes at 121°C. The supernatant was decanted into a container and clarified by either centrifugation or microfiltration. To the clarified supernatant, 1 N HCl was added at a rate of 2 ml per 100 ml extract. The extract was stirred for at least fifteen minutes at room temperature and then clarified as described above. The clarified extract was sterilized by autoclaving as described above or by microfiltration.

To the pretreated broth the following media components were added: 0.01% thallium acetate; 0.005% ampicillin; 0.0125% cysteine hydrochloride; 6.25% yeast extract, 1% dextrose; 10% swine serum (Gibco) heat inactivated; and, optionally, 0.0026% phenol red. The pH of the culture medium was adjusted to pH 7.5 ± 0.2 and filter sterilized.

To initiate a production serial, frozen M. hyopneumoniae master seed was thawed and a 5-20% suspension inoculated into 100-3000 ml of the culture medium described above. The culture was incubated at 30°C to 39°C for 36 to 168 hours. Following satisfactory growth, the culture was transferred into a seeding container with fresh medium, using a 5-20% inoculum. This culture was incubated at 37°C ± 1°C for 36 to 96 hours.

Production cultures of M. hyopneumoniae are grown in fermentors, incubated at 37°C ± 1°C for 36 to 96 hours following inoculation. The dissolved oxygen content of the culture is maintained at between 20-40% by aeration with sterile air and agitation. Sterile antifoam may be used to control foam.

At the end of the growth period, the pH of the culture was raised to 7.6 ± 0.2 and the pH maintained in this range for about one hour. To inactivate the organism, a filter-sterilized aqueous solution of 2-bromoethylaminehydrobromide (BEA) was added to a final concentration of approximately 4.0 mM. BEA is converted to the inactivating agent binary ethyleneimine (BEI) at the increased pH of the culture. The culture was incubated at 37°C ± 1°C with constant agitation for at least 24 hours.

After the 24 hour incubation, a filter sterilized aqueous solution of sodium thiosulfate, a standard neutralizing agent, was added to a final concentration of approximately 4 mM to neutralize excess BEI. The culture was incubated for an additional 24 hours at 37°C ± 1°C to complete inactivation.

### EXAMPLE 2

### Preparation of a Vaccine

Following inactivation of the vaccine component of Example 1, a vaccine was formulated by adding to the inactivated M. hyopneumoniae several conventional vaccine components. Sufficient inactivated M. hyopneumoniae was combined with phosphate buffered saline diluent to obtain a minimum antigen concentration of 5 X 10⁸ CCU and a maximum of 5 X 10⁹ CCU of M. hyopneumoniae per 2 ml dose. Sterile 10% merthiolate and 10% ethylenediamine tetra acetic acid (EDTA, disodium or tetrasodium salt) solutions were added as preservatives. Sterile mineral oil [Drakeol] containing 5% to 40% by weight of lecithin (Central Soya) was added as an adjuvant. The final concentration of between 0.7% to 3.2% Tween 80 and 0.3% to 1.8% Span was added as an emulsifier. Selected parabens (methyl p-hydroxylbenzoate, propyl p-hydroxylbenzoate, butyl p-hydroxylbenzoate) may be added as additional preservatives for the oil and emulsifiers.

### EXAMPLE 3

### Vaccine Challenge Experiments

Two separate vaccination-challenge experiments were conducted to evaluate the protective capabilities of an inactivated, adjuvanted M. hyopneumoniae vaccine in swine.

Thirty-three, crossbred, six and one-half week old pigs were obtained from a closed, respiratory disease-free herd and allocated to one nonvaccinated challenged control group (Group 1), two vaccinated challenge groups (Groups 2 and 3), and one nonvaccinated nonchallenged control group (Group 4).

Animals in Group 2 received the vaccine described in Example 1 except that the M. hyopneumoniae culture was inactivated with 0.3% formalin instead of BEI; and animals in Group 3 received the vaccine described in Example 1, in both cases administered in 2 ml doses intramuscularly on day "O" and day 14. Both vaccines contained inactivated whole cells adjuvanted the same way, differing only with respect to the inactivating agent used. Pigs in Group 4 received 6 ml doses of incomplete Freund adjuvanted Friis mycoplasma broth (placebo) via the same route.

Pneumonia was induced by intratracheal inoculation of pigs [Bentley, O.E. and Farrington, D.O. 1980. Am. J. Vet. Res. 41:1870] seven days after the second dose of vaccine with a single 10 ml dose of crude lung homogenate containing M. hyopneumoniae strain 232 [derived from strain 11]. Pigs in Group 4 were given 10 ml. Friis mycoplasma broth.

Necropsy was performed approximately 3 weeks post-challenge. Criteria utilized for determination of efficacy of vaccines for prophylaxis of M. hyopneumoniae disease were (a) severity of clinical signs; (b) macroscopic lesions of pneumonia; (c) microscopic lesions typical of the disease; and (d) infection of lung tissue determined by immunofluorescence [Amaneu, W. et al. Proceedings, IPVS Congress, Copenhagen, Denmark. p. 223 (1980)].

As shown in Tables 1 and 2 below, pigs in Groups 1 and 2 tended to have slightly higher coughing scores than pigs in Groups 3 and 4. All pigs in Group 1 (positive control) and most pigs in Groups 2 and 3 (vaccinated) had lesions. None of the pigs in Group 4 had lesions. Numbers of lobes with lesions were substantially less in Group 3 pigs than pigs in Groups 1 and 2. Most importantly, mean percent of lungs with pneumonia and mean lung lesion scores were significantly less in vaccinated Group 3 than in Group 1. Severity of pneumonia in Group 2 was not significantly different from Group 1 (Table 1).

Most pigs in all groups had some microscopic lesions. Severity of microscopic lesions were significantly less in Groups 2, 3 and 4 than in positive Group 1.

Evaluation of lungs by immunofluorescence revealed that 7 out of 8 pigs in Group 1 were positive for the disease. Only four in ten pigs and three in nine pigs were FA positive in vaccinated groups 2 and 3, respectively.

All pigs in Groups 2 and 3 had developed high complement fixing (CF) antibody titers to M. hyopneumoniae by the time they were challenged. A more rapid development of CF antibody positive status was observed with Group 2 than with Group 3.

In summary, the vaccine administered in Group 3 pigs provided relatively strong protection against intratracheal challenge with M. hyopneumoniae lung homogenate. Protection was evidenced by the reduced number of lobes with lesions, reduced percentage of lungs with lesions, reduced mean lung lesion scores, reduced microscopic lesion scores, and reduced number of pigs FA positive.

**Table 1**

| Groups | 1 | 2 | 3 | 4 |
|---|---|---|---|---|
| No. of pigs | 8 | 10 | 9 | 6 |
| Vaccine | - | XMHP4 | XMHP5 | Placebo |
| Mean Coughing Score ± S.D. | 1.11 ± 0.11 | 1.13± 0.15 | 1.05± 0.10 | 1.01±0.02 |

| Gross Lesions | | | | |
|---|---|---|---|---|
| No. with lesions | 8 | 7 | 6 | 0 |
| No. lobes positive | 35/56 | 23/70 | 12/63 | 0/42 |
| Mean % lung with lesions ± S. D. | 7.93± 4.5 | 6.80± 7.73 | 1.9± 3.39 | 0 |

| Microscopic lesions | | | | |
|---|---|---|---|---|
| No. with lesions | 8 | 7 | 8 | 4 |
| Mean lesion scores ± S.D. | 2.81± 0.40 | 2.0± 0.84 | 1.72± 0.68 | 1.29± 0.25 |

| Immunofluorescence | | | | |
|---|---|---|---|---|
| No. positive | 7 | 4 | 3 | 0 |
| Avg. score | 0.63 | 0.28 | 0.18 | 0 |
| CF Antibody Titers pre-challenge | <4 | 2521 | 474 | <4 |

In the second experiment, the efficacy of the inactivated vaccine of Example 1 was confirmed in piglets (conventionally raised), vaccinated at 1 and 3 weeks of age. Experimental design and results are summarized in Table 2.

**Table 2**

| Study II | Groups | A | B |
|---|---|---|---|
| No. of pigs | | 38 | 29 |
| Dosage/Route Challenge | | 2 ml IM-vaccine Intranasal | 2ml IM-placebo Intranasal |
| No. pigs challenged with % Mean lesion* scores | NL1042 | 25 | 23 |
| | ISU232 | 13 | 6 |
| | NL1042 | 4.98 | 27.91 |
| | ISU232 | 1.82 | 19.24 |

| | | | |
|---|---|---|---|
| *Method of Goodwin and Whittlestone | | | |

### EXAMPLE 4

### Preparing Pasteurella Multocida Toxoid for a Combination Vaccine

An additional vaccine component for a combination vaccine of this invention may include the following P. multocida toxoid.

### A. Culturing the P. multocida

P. multocida type D (strain 8) [Dr. Ross Cowart, University of Illinois, Urbana, Illinois] is subcultured in a modified chemically defined synthetic medium for one day. The medium is described by Herriott et al, J. Bact., 101:513-516 (1970).

The pH of the assembled medium is adjusted to 7.3 ± 0.2 with sterile NaOH. Cells from this culture are transferred to fresh synthetic medium and this culture, when grown, is combined with a cryopreservative and stored at -70°C. Production cultures are grown to harvest during incubation at approximately 36° ± 1°C for between 3 and 24 hours following inoculation. The dissolved oxygen content of the culture is maintained by aeration with sterile air and by agitation. Sterile antifoam solution is used to control foam. The pH of the culture is maintained at 7.3 ± 0.2.

At the end of the growth cycle, P. multocida cultures are examined and cell density is determined by absorbance at 650 nm. Agitation is then decreased, and aeration and pH control are discontinued.

### B. Pre-detoxification treatment

Following growth of the organism, sterile merthiolate is added to the culture in an amount less than or equal to 0.01 percent weight per volume. Culture fluids may be aseptically transferred through closed connections to a sterile closed container. The container is connected through closed fittings to an apparatus used to physically lyse cells and release cellular contents, e.g., a "GAULIN" model 15M laboratory homogenizer.

Bacterial cells in the culture fluid are lysed by continuous passage through the pressure chamber of the homogenizer. This subjects the cells to an immediate pressure drop from between an initial pressure of between 13790 kPa (2000 psi) and 34475 kPa (5000 psi) to ambient pressure of 103.4 kPa (15 psi). The lysed cells are aseptically deposited into another closed container.

The lysate is clarified by sequential steps of centrifugation and/or microporous filtration. Clarified solutions may be concentrated before or after filter sterilization. Ethylenediaminetetraacetic acid (EDTA), in an amount up to a final concentration of 5 mM, and glycerol, in an amount up to a final concentration of 1.0% (vol/vol), are added before concentrating and filter-sterlizing, to prevent aggregation of the concentrated proteins.

### C. Detoxification

Detoxification is achieved by the following process. Sterile 5 N NaOH is slowly and aseptically added to sterile toxin to increase the pH from its initial level of a pH of approximately 7.0 to a pH of approximately 10.55 ± 0.10. The pH is maintained at this level for approximately 7 hours. Thereafter the pH is adjusted to 7.0 ± 0.2 by slowly and aseptically adding sterile 4 N HCl. Fluids are held at this pH for 1 hour. Agitation and temperature are maintained at a constant level throughout the process.

Preferably this detoxification process is performed a total of 3 times within 25 hours or until detoxification is complete. The toxoid is then stored at 2° to 7°C until combined with other components and assembled into vaccine compositions.

Aliquots are taken each time the pH is approximately 7.0, e.g., at the starting pH and whenever the pH is adjusted to approximately 7. Residual toxicity of each aliquot is measured and expressed in mouse LD₅₀'S per mL. A preparation with an initial value of nearly 2,500 LD₅₀'s per mL is usually completely detoxified approximately 25 hours after the pH is first adjusted to 10.55, without appreciable decrease in assayable antigen content.

### EXAMPLE 5

### Preparing a P. Multocida Bacterin-Toxoid Vaccine Component

A combination vaccine of this invention may include a bacterin-toxoid of P. multocida in which the toxoid has been stabilized within the bacterial cell.

A culture of P. multocida, type D, strain 4677, is grown in the following medium: Tryptic Soy Broth without Dextrose (Difco) 30 g; Yeast extract (Difco) 5 g; Dextrose 4 g; Deionized water to 1 liter; pH of approximately 7; sterilized by autoclaving at 121°C.

The culture is aerated with agitation to maintain the dissolved oxygen concentration at approximately 35% of saturation. The temperature is maintained at 37°C, and the pH at 7 by the addition of 10N NaOH solution as needed. Towards the end of exponential growth, aeration is discontinued and the culture is inactivated by the addition of formaldehyde solution (USP) to a final concentration of 0.5% v/v. The culture is then held at 37°C for four days. Other inactivating agents, such as beta-propriolactone, glutaraldehyde, and binary ethyleneamine can be used in place of formaldehyde.

A sample is withdrawn to test whether inactivation is complete by administering the sample to guinea pigs. Guinea pigs should be alive and healthy at 7 days after subcutaneous injection with 4 ml volumes of the culture. At this point the toxin within the cells is completely converted to toxoid, which is safe, very stable and capable of inducing the production of neutralizing antitoxins upon injection into animals.

The inactivated culture is centrifuged. The sedimented bacteria are dispensed in sufficient supernatant fluid to make a suspension with an OD (optical density at 625 nm, as determined in a Spectronic 20 spectrophotometer) of 4.2. The suspension is then adsorbed with Al(OH)₃ gel, 25% v/v, thimerosol (0.01% w/v) is added as a preservative, and the pH is adjusted to 6.5 ± 0.2.

### EXAMPLE 6

### Co-administration Experiments with Two Vaccines

To determine if conventional swine vaccines containing Bordetella bronchiseptica, Erysipelothrix rhusipathiae, and P. multocida whole cell vaccines, type A and D, would adversely be affected by the vaccine containing the inactivated M. hyopneumoniae component of Example 1 a study was conducted. Both vaccines were simultaneously administered by the same route at different sites.

The M. hyopneumoniae vaccine referred to above evidenced no immunosuppressive effects or other adverse interference on the animals' response to the other vaccine as measured by seroconversion responses.

Alternatively, the M. hyopneumoniae bacterin of this invention may be employed in vaccine compositions with such other vaccine components.

Numerous modifications and variations of the present invention are included in the above-identified specification and are expected to be obvious to one of skill in the art. For example, use of other appropriate strains of M. hyopneumoniae, or strains for combination vaccines, and vaccine components, such as adjuvants, preservatives and the like, may be selected by one of skill in the art. Such modifications and alterations to the compositions and processes of the present invention are believed to be encompassed in the scope of the claims appended hereto.

## Claims (Claims for the following Contracting State(s): AT, BE, CH, LI, DE, DK, FR, GB, IT, LU, NL, SE)

1. A vaccine component comprising M. hyopneumoniae inactivated with binary ethyleneimine at a dosage of at least 5 x 10⁸ CCU, said component being capable of inducing an immunological response in vaccinated swine against M. hyopneumoniae.

2. The vaccine component according to claim 1 produced by pretreating a culture medium capable of sustaining M. hyopneumoniae by anion exchange resin; inoculating said pretreated medium with M. hyopneumoniae, increasing the dissolved oxygen content of said culture to between 20 to 40% of saturation, culturing the M. hyopneumoniae to a titer of at least 1 x 10⁸ CCU, and inactivating the culture by the addition of binary ethyleneimine.

3. The component according to claim 2 comprising a titer of 5 x 10⁸ to 5 x 10⁹ color changing units.

4. The component according to claim 2 wherein said culturing step comprises growing the culture of M. hyopneumoniae at a temperature of between 30° to 40°C at neutral to slightly alkaline pH.

5. A vaccine capable of inducing immunity to M. hyopneumoniae in a mammal without serious side effects comprising a vaccine amount of the component of claim 1 and an adjuvant to elicit an immunoprotective response in a porcine animal, the component having immunogenic activity in at least an amount sufficient to protect the animal against challenge by M. hyopneumoniae.

6. The vaccine according to claim 5 wherein the component comprises the inactivated M. hyopneumoniae at a titer of between 5 X 10⁸ to 5 X 10¹⁰ color changing units.

7. The vaccine according to claim 5 wherein said adjuvant is selected from the group consisting of: lecithin and mineral oil, saponins, aluminum hydroxide.

8. A method for the preparation of a vaccine for protecting mammals against M. hyopneumoniae which comprises pretreating a culture medium capable of sustaining M. hyopneumoniae by anion exchange resin, inoculating said medium with M. hyopneumoniae, increasing the dissolved oxygen content of said culture to between 20 to 40% of saturation, culturing the M. hyopneumoniae to a titer of at least 1 X 10⁸ CCU, and inactivating the culture by the addition of binary ethyleneimine.

9. The method according to claim 8 wherein said titer is between 5 X 10⁸ to 5 X 10¹⁰ color changing units.

10. The method according to claim 8 wherein said culturing step comprises growing the culture of M. hyopneumoniae at a temperature of between 30° to 40°C at neutral to slightly alkaline pH.

11. Use of a vaccinal amount of a vaccine component comprising M. hyopneumoniae inactivated with binary ethyleneimine at a dosage of at least 5 X 10⁸ CCU, said component being capable of inducing an immunological response in vaccinated swine against M. hyopneumoniae and an adjuvant for the preparation of a vaccine for vaccinating pigs against M. hyopneumoniae.

12. A vaccine composition comprising an inactivated M. hyopneumoniae vaccine component and an immunogenic amount of one or more additional antigens, wherein said additional antigens are selected from the group consisting of a Pasteurella multocida bacterin with a cell-bound toxoid, a Bordetella bronchiseptica bacterin, an Erysipelothrix rhusiopathiae antigen extract, a soluble cell-free toxoid of Pasteurella multocida type D, inactivated whole cells of P. multocida type A or D, cultures of Actinobacillus pleuropneumoniae Haemophilus parasuis, and Pseudorabies virus.

13. A vaccine composition according to claim 12 consisting of inactivated M. hyopneumoniae, inactivated Bordetella bronchiseptica, inactivated P. multocida type A, inactivated P. multocida type D, P. multocida cell-free toxoid and Erysipelothrix rhusiopathiae antigen extract.

14. A vaccine composition according to claim 12 consisting of inactivated M. hyopneumoniae, and vaccine components of Actinobacillus pleuropneumoniae, Haemophilus parasuis, and Pseudorabies virus.

15. Use of an inactiviated M. hyopneumoniae vaccine component and an immunogenic amount of one or more additional antigens, wherein said additional antigens are selected from the group consisting of a Pasteurella multocida bacterin with a cell-bound toxoid, a Bordetella bronchiseptica bacterin, an Erysipelothrix rhusiopathiae antigen extract, a soluble cell-free toxoid of Pasteurella multocida type D, inactivated whole cells of P. multocida type A or D, cultures of Actinobacillus pleuropneumoniae, Haemophilus parasuis, and Pseudorabies virus, for the preparation of a vaccine for vaccinating a pig against M. hyopneumoniae and secondary bacterial infections.

16. Use of inactivated M. hyopneumoniae, inactivated Bordetella bronchiseptica, inactivated P. multocida type A, inactivated P. multocida type D, and P. multocida cell-free toxoid, and Erysipelothrix rhusiopathiae antigen extract for the preparation of a vaccine composition for vaccinating a pig against M. hyopneumoniae and secondary bacterial infections.

17. Use of M. hyopneumoniae inactivated with binary ethyleneimine at a dosage of at least 5 X 10⁸ CCU and an adjuvant for the preparation of a vaccine and additional antigen for the preparation of one or more vaccines for protecting swine against M. hyopneumoniae infection and other pathogenic infections, wherein said vaccines are to be administered to an animal sequentially or simultaneously.

18. Use according to claim 17, wherein said additional antigen is selected from the group consisting of a Pasteurella multocida bacterin with a cell-bound toxoid, a Bordetella bronchiseptica bacterin, an Erysipelothrix rhusiopathiae antigen extract, a soluble cell-free toxoid of Pasteurella multocida type D, inactivated whole cells of P. multocida type A or D, cultures of Actionbacillus pleuropneumoniae, Haemophilus parasuis, and Pseudorabies virus for the preparation of a vaccine for vaccinating a pig against M. hyopneumoniae and secondary bacterial infections.

## Claims (Claims for the following Contracting State(s): ES, GR)

1. A method of producing a vaccine capable of inducing immunity to M. hyopneumoniae in a mammal without serious side effects comprising combining a vaccinal amount of a vaccine component comprising M. hyopneumoniae inactivated with binary ethyleneimine at a dosage of at least 5 X 10⁸ CCU, said component being capable of inducing an immunological response in vaccinated swine against M. hyopneumoniae and an adjuvant.

2. The method according to claim 1 wherein the component comprises the inactivated M. hyopneumoniae at a titer of between 5 X 10⁸ to 5 X 10¹⁰ color changing units.

3. The method according to claim 1, wherein said adjuvant is selected from the group consisting of: lecithin and mineral oil, saponins, aluminum hydroxide.

4. A method for the preparation of a vaccine for protecting mammals against M. hyopneumoniae which comprises pretreating a culture medium capable of sustaining M. hyopneumoniae by anion exchange resin, inoculating said medium with M. hyopneumoniae, increasing the dissolved oxygen content of said culture to between 20 to 40% of saturation, culturing the M. hyopneumoniae to a titer of at least 1 X 10⁸ CCU, and inactivating the culture by the addition of binary ethyleneimine.

5. The method according to claim 4, wherein said titer is between 5 X 10⁸ to 5 X 10¹⁰ color changing units.

6. The method according to claim 4, wherein said culturing step comprises growing the culture of M. hyopneumoniae at a temperature of between 30° to 40°C at neutral to slightly alkaline pH.

7. A method of producing a vaccine composition comprising combining an inactivated M. hyopneumoniae vaccine component and an immunogenic amount of one or more additional antigens, wherein said additional antigens are selected from the group consisting of a Pasteurella multocida bacterin with a cell-bound toxoid, a Bordetella bronchiseptica bacterin, an Erysipelothrix rhusiopathiae antigen extract, a soluble cell-free toxoid of Pasteurella multocida type D, inactivated whole cells of P. multocida type A or D, cultures of Actinobacillus pleuropneumoniae, Haemophilus parasuis, and Pseudorabies virus.

8. The method of claim 7, wherein said vaccine composition consists of inactivated M. hyopneumoniae, inactivated Bordetella bronchiseptica, inactivated P. multocida type A, inactivated P. multocida type D, P. multocida cell-free toxoid, and Erysipelothrix rhusiopathiae antigen extract.

9. The method of claim 7, wherein said vaccine composition consists of inactivated M. hyopneumoniae, and vaccine components of Actinobacillus pleuropneumoniae, Haemophilus parasuis, and Pseudorabies virus.

10. The method of claim 7, wherein the vaccine composition is to be administered to an animal sequentially or simulaneously.

## Patentansprüche (Patentansprüche für folgende(n) Vertragsstaat(en): AT, BE, CH, LI, DE, DK, FR, GB, IT, LU, NL, SE)

1. Impfstoffbestandteil, der durch binäres Ethylenimin inaktivierten M. hyopneumoniae in einer Dosierung von mindestens 5 x 10⁸ CCU umfasst und zur Induktion einer immunologischen Antwort in einem geimpften Schwein gegen M. hyopneumoniae fähig ist.

2. Impfstoffbestandteil nach Anspruch 1, der hergestellt wird durch Vorbehandlung eines Kulturmediums, in dem M. hyopneumoniae erhalten werden kann, mit einem Anionenaustauschharz, Inokulation des vorbehandelten Mediums mit M. hyopneumoniae, Erhöhung des Gehalts der Kultur an gelöstem Sauerstoff auf 2 bis 40% Sättigung, Züchtung von M. hyopneumoniae bis zu einem Titer von mindestens 1 x 10⁸ CCU und Inaktivierung der Kultur durch Zugabe von binärem Ethylenimin.

3. Bestandteil nach Anspruch 2, der einen Titer von 5 x 10⁸ bis 5 x 10⁹ farbverändernden Einheiten umfasst.

4. Bestandteil nach Anspruch 2, wobei der Züchtungsschritt das Wachstum der Kultur von M. hyopneumoniae bei einer Temperatur von 30°C bis 40°C bei neutralem bis schwach alkalischem pH-Wert umfasst.

5. Impfstoff, der eine Immunität gegen M. hyopneumoniae in einem Säuger ohne ernste Nebeneffekte induzieren kann, umfassend eine zur Impfung geeignete Menge des Bestandteils von Anspruch 1 und ein Adjuvans zum Auslösen einer immunprotektiven Antwort in einem Schwein, wobei der Bestandteil eine immunogene Aktivität in mindestens einer Menge aufweist, die zum Schutz des Tieres bei Exposition gegen M. hyopneumoniae ausreichend ist.

6. Impfstoff nach Anspruch 5, wobei der Bestandteil den inaktivierten M. hyopneumoniae in einem Titer von 5 x 10⁸ bis 5 x 10¹⁰ farbverändernden Einheiten umfasst.

7. Impfstoff nach Anspruch 5, wobei das Adjuvans ausgewählt ist aus der Gruppe bestehend aus: Lecithin und Mineralöl, Saponine und Aluminiumhydroxid.

8. Verfahren zur Herstellung eines Impfstoffes zum Schutz von Säugern gegen M. hyopneumoniae, umfassend die Vorbehandlung eines Kulturmediums, in dem M. hyopneumoniae erhalten werden kann, mit einem Anionenaustauschharz, die Inokulation des Mediums mit M. hyopneumoniae, die Erhöhung des Gehalts der Kultur an gelöstem Sauerstoff auf 20 bis 40% Sättigung, die Züchtung von M. hyopneumoniae bis zu einem Titer von mindestens 1 x 10⁸ CCU und die Inaktivierung der Kultur durch Zugabe von binärem Ethylenimin.

9. Verfahren nach Anspruch 8, wobei der Titer bei 5 x 10⁸ bis 5 x 10¹⁰ farbverändernden Einheiten liegt.

10. Verfahren nach Anspruch 8, wobei der Züchtungsschritt das Wachstum der Kultur von M. hyopneumoniae bei einer Temperatur von 30°C bis 40°C bei neutralem bis schwach alkalischem pH-Wert umfasst.

11. Verwendung einer zur Impfung geeigneten Menge eines Impfstoffbestandteils, umfassend den mit binärem Ethylenimin inaktivierten M. hyopneumoniae in einer Dosierung von mindestens 5 x 10⁸ CCU, wobei der Bestandteil eine immunologische Antwort in einem geimpften Schwein gegen M. hyopneumoniae induzieren kann, und ein Adjuvans für die Herstellung eines Impfstoffes zur Impfung von Schweinen gegen M. hyopneumoniae.

12. Impfstoffzusammensetzung, die einen Impfstoffbestandteil aus inaktiviertem M. hyopneumoniae und eine immunogene Menge von einem oder mehreren zusätzlichen Antigenen umfasst, wobei die zusätzlichen Antigene ausgewählt sind aus der Gruppe bestehend aus einem Pasteurella multocida-Bacterin mit einem zellgebundenen Toxoid, einem Bordetella bronchiseptica-Bacterin, einem Erysipelothrix rhusiopathiae-Antigenextrakt, einem löslichen zellfreien Toxoid von Pasteurella multocida Typ D, inaktivierten ganzen Zellen von P. multocida Typ A oder D, Kulturen von Actinobacillus pleuropneumoniae, Haemophilus parasuis und Pseudorabies-Virus.

13. Impfstoffzusammensetzung nach Anspruch 12, die aus inaktiviertem M. hyopneumoniae, inaktiviertem Bordetella bronchiseptica, inaktiviertem P. multocida Typ A, inaktiviertem P. multocida Typ D, zellfreiem Toxoid von P. multocida und Antigenextrakt von Erysipelothrix rhusiopathiae besteht.

14. Impfstoffzusammensetzung nach Anspruch 12, die aus inaktiviertem M. hyopneumoniae und Impfstoffbestandteilen von Actinobacillus pleuropneumoniae, Haemophilus parasuis und Pseudorabies-Virus besteht.

15. Verwendung eines Impfstoffbestandteils aus inaktiviertem M. hyopneumoniae und einer immunogenen Menge von einem oder mehreren zusätzlichen Antigenen, wobei die zusätzlichen Antigene ausgewählt sind aus der Gruppe bestehend aus einem Pasteurella multocida-Bacterin mit einem zellgebundenen Toxoid, einem Bordetella bronchiseptica-Bacterin, einem Erysipelothrix rhusiopathiae-Antigenextrakt, einem löslichen zellfreien Toxoid von Pasteurella multocida Typ D, inaktivierten ganzen Zellen von P. multocida Typ A oder D, Kulturen von Actinobacillus pleuropneumoniae, Haemophilus parasuis und Pseudorabies-Virus, für die Herstellung eines Impfstoffes zur Impfung eines Schweins gegen M. hyopneumoniae und sekundäre bakterielle Infektionen.

16. Verwendung von inaktiviertem M. hyopneumoniae, inaktiviertem Bordetella bronchiseptica, inaktiviertem P. multocida Typ A, inaktiviertem P. multocida Typ D, zellfreiem Toxoid von P. multocida und Antigenextrakt von Erysipelothrix rhusiopathiae für die Herstellung einer Impfstoffzusammensetzung zur Impfung eines Schweins gegen M. hyopneumoniae und sekundäre bakterielle Infektionen.

17. Verwendung von mit binärem Ethylenimin inaktiviertem M. hyopneumoniae in einer Dosierung von mindestens 5 x 10⁸ CCU und einem Adjuvans für die Herstellung eines Impfstoffes und zusätzlichem Antigen für die Herstellung von einem oder mehreren Impfstoffen zum Schutz eines Schweins gegen eine M. hyopneumoniae-Infektion und andere pathogene Infektionen, wobei die Impfstoffe sequentiell oder gleichzeitig an ein Tier verabreicht werden sollen.

18. Verwendung nach Anspruch 17, wobei das zusätzliche Antigen ausgewählt ist aus der Gruppe bestehend aus einem Pasteurella multocida-Bacterin mit einem zellgebundenen Toxoid, einem Bordetella bronchiseptica-Bacterin, einem Antigenextrakt von Erysipelothrix rhusiopathiae, einem löslichen zellfreien Toxoid von Pasteurella multocida Typ D, inaktivierten ganzen Zellen von P. multocida Typ A oder D, Kulturen von Actinobacillus pleuropneumoniae, Haemophilus parasuis und Pseudorabies-Virus, für die Herstellung eines Impfstoffes zur Impfung eines Schweins gegen M. hyopneumoniae und sekundäre bakterielle Infektionen.

## Patentansprüche (Patentansprüche für folgende(n) Vertragsstaat(en): ES, GR)

1. Verfahren zur Herstellung eines Impfstoffes, der eine Immunität gegen M. hyopneumoniae in einem Säuger ohne ernste Nebeneffekte induzieren kann, umfassend die Formulierung einer zur Impfung geeigneten Menge eines Impfstoffbestandteils, der durch binäres Ethylenimin inaktivierten M. hyopneumoniae umfasst, in einer Dosierung von mindestens 5 x 10⁸ CCU, wobei der Bestandteil zur Induktion einer immunologischen Antwort in einem geimpften Schwein gegen M. hyopneumoniae fähig ist, und ein Adjuvans.

2. Verfahren nach Anspruch 1, wobei der Bestandteil den inaktivierten M. hyopneumoniae in einem Titer von 5 x 10⁸ bis 5 x 10¹⁰ farbverändernden Einheiten umfasst.

3. Verfahren nach Anspruch 1, wobei das Adjuvans ausgewählt ist aus der Gruppe bestehend aus: Lecithin und Mineralöl, Saponine und Aluminiumhydroxid.

4. Verfahren zur Herstellung eines Impfstoffes zum Schutz von Säugern gegen M. hyopneumoniae, umfassend die Vorbehandlung eines Kulturmediums, in dem M. hyopneumoniae erhalten werden kann, mit einem Anionenaustauschharz, Inokulation des Mediums mit M. hyopneumoniae, Erhöhung des Gehalts der Kultur an gelöstem Sauerstoff auf 20 bis 40 % Sättigung, Züchtung von M. hyopneumoniae bis zu einem Titer von mindestens 1 x 10⁸ CCU und Inaktivierung der Kultur durch Zugabe von binärem Ethylenimin.

5. Verfahren nach Anspruch 4, wobei der Titer bei 5 x 10⁸ bis 5 x 10¹⁰ farbverändernden Einheiten liegt.

6. Verfahren nach Anspruch 4, wobei der Züchtungsschritt das Wachstum der Kultur von M. hyopneumoniae bei einer Temperatur von 30°C bis 40°C bei neutralem bis schwach alkalischem pH-Wert umfasst.

7. Verfahren zur Herstellung einer Impfstoffzusammensetzung, die das Kombinieren einer Impfstoffkomponente aus inaktiviertem M. hyopneumoniae und einer immunogenen Menge von einem oder mehreren zusätzlichen Antigenen umfasst, wobei die zusätzlichen Antigene ausgewählt sind aus der Gruppe bestehend aus einem Pasteurella multocida-Bacterin mit einem zellgebundenen Toxoid, einem Bordetella bronchiseptica-Bacterin, einem Erysipelothrix rhusiopathiae-Antigenextrakt, einem löslichen zellfreienToxoid von Pasteurella multocida Typ D, inaktivierten ganzen Zellen von P. multocida Typ A oder D, Kulturen von Actinobacillus pleuropneumoniae, Haemophilus parasuis und Pseudorabies-Virus.

8. Verfahren nach Anspruch 7, wobei die Impfstoffzusammensetzung aus inaktiviertem M. hyopneumoniae, inaktiviertem Bordetella bronchiseptica, inaktiviertem P. multocida Typ A, inaktiviertem P. multocida Typ D, zellfreiem Toxoid von P. multocida und Antigenextrakt von Erysipelothrix rhusiopathiae besteht.

9. Verfahren nach Anspruch 7, wobei die Impfstoffzusammensetzung aus inaktiviertem M. hyopneumoniae und Impfstoffbestandteilen von Actinobacillus pleuropneumoniae, Haemophilus parasuis und Pseudorabies-Virus besteht.

10. Verfahren nach Anspruch 7, wobei die Impfstoffzusammensetzung sequentiell oder gleichzeitig an ein Tier verabreicht werden soll.

## Revendications (Revendications pour l'(les) Etat(s) contractant(s) suivant(s): AT, BE, CH, LI, DE, DK, FR, GB, IT, LU, NL, SE)

1. Composant de vaccin comprenant *M. hyopneumoniae* inactivé avec de l'éthylène imine binaire à une posologie d'au moins 5 x 10⁸ CCU, ledit composant étant capable d'induire une réponse immunologique chez le porc vacciné contre *M. hyopneumoniae*.

2. Composant de vaccin selon la revendication 1, produit par le prétraitement d'un milieu de culture capable de soutenir *M. hyopneumoniae*, par une résine d'échange d'anions, l'inoculation dudit milieu prétraité avec *M*. *hyopneumoniae*, l'augmentation de la teneur en oxygène dissout de ladite culture jusqu'à une saturation comprise entre 20 et 40 %, la culture de *M. hyopneumoniae* jusqu'à un titre d'au mois 1 x 10⁸ CCU et l'inactivation de la culture par l'ajout d'éthylène imine binaire.

3. Composant selon la revendication 2, comprenant un titre de 5 x 10⁸ à 5 x 10⁹ unités de changement de couleur (color changing units).

4. Composant selon la revendication 2, dans lequel ladite étape de culture comprend la mise en croissance de la culture de *M. hyopneumoniae* à une température comprise entre 30 °C et 40 °C, à un pH allant de neutre à légèrement alcalin.

5. Vaccin capable d'induire l'immunité contre *M. hyopneumoniae* chez un mammifère sans effets secondaires graves, comprenant une quantité vaccinale du composant selon la revendication 1 et un adjuvant pour provoquer une réponse immunoprotectrice chez un animal porcin, le composant ayant une activité immunogène dans au moins une quantité suffisante pour protéger l'animal contre un test de provocation par *M*. *hyopneumoniae*.

6. Vaccin selon la revendication 5, dans lequel le composant comprend le *M. hyopneumoniae* inactivé à un titre compris entre 5 x 10⁸ à 5 x 10¹⁰ unités de changement de couleur (color changing units).

7. Vaccin selon la revendication 5, dans lequel ledit adjuvant est choisi dans le groupe constitué de : la lécithine et l'huile minérale, les saponines, l'hydroxyde d'aluminium.

8. Procédé pour la préparation d'un vaccin pour la protection des mammifères contre *M. hyopneumoniae*, qui comprend le prétraitement d'un milieu de culture capable de soutenir *M. hyopneumoniae* par une résine d'échange d'anions, l'inoculation dudit milieu avec *M. hyopneumoniae*, l'augmentation de la teneur en oxygène dissout de la dite culture jusqu'à une saturation comprise entre 20 et 40 %, la mise en culture de *M. hyopneumoniae* jusqu'à un titre d'au moins 1 x 10⁸ CCU et l'inactivation de la culture par l'ajout d'éthylène imine binaire.

9. Procédé selon la revendication 8, dans lequel ledit titre est compris entre 5 x 10⁸ à 5 x 10¹⁰ unités de changement de couleur (color changing units).

10. Procédé selon la revendication 8, dans lequel ladite étape de culture comprend la mise en croissance de la culture de *M. hyopneumoniae* à une température comprise entre 30 °C et 40 °C à un pH allant de neutre à légèrement alcalin.

11. Utilisation d'une quantité vaccinale d'un composant de vaccin comprenant *M. hyopneumoniae* inactivé avec de l'éthylène imine binaire à une posologie d'au moins 5 x 10⁸ CCU, ledit composé étant capable d'induire une réponse immunologique chez le porc vacciné contre *M. hyopneumoniae* et un adjuvant pour la préparation d'un vaccin pour la vaccination des cochons contre *M. hyopneumoniae*.

12. Composition vaccinale comprenant un composant de vaccin de *M. hyopneumoniae* inactivé et une quantité immunogène d'un ou plusieurs antigènes supplémentaires, dans laquelle lesdits antigènes supplémentaires sont choisis dans le groupe constitué d'une bactérine de *Pasteurella multocida* avec un toxoïde fixé aux cellules, d'une bactérine de *Bordetella bronchiseptica*, d'un extrait d'antigène d' *Erysipelothrix rhusiopathiae*, d'un toxoïde acellulaire soluble de *Pasteurella multocida* de type D, de cellules entières inactivées de *P. multocida* de type A ou D, de cultures d'*Actinobacillus pleuropneumoniae*, d'*Haemophilus parasuis* et de virus de la pseudo-rage.

13. Composition vaccinale selon la revendication 12, constituée de *M. hyopneumoniae* inactivé, de *Bordetella bronchiseptica* inactivé, de *P. multocida* de type A inactivé, de *P. multocida* de type D inactivé, d'un toxoïde acellulaire de *P*. *multocida* et d'un extrait d'antigène d'*Erysipelothrix rhusiopathiae*.

14. Composition vaccinale selon la revendication 12, constituée de *M. hyopneumoniae* inactivé et de composants de vaccin d'*Actinobacillus pleuropneumoniae,* d'*Haemophilus parasuis* et de virus de la pseudo-rage.

15. Utilisation d'un composant de vaccin de *M. hyopneumoniae* inactivé et d'une quantité immunogène d'un ou plusieurs antigènes supplémentaires, dans laquelle lesdits antigènes supplémentaires sont choisis dans le groupe constitué d'une bactérine de *Pasteurella multocida* avec un toxoïde fixé aux cellules, d'une bactérine de *Bordetella bronchiseptica*, d'un extrait d' antigène d' *Erysipelothrix rhusiopathiae*, d'un toxoïde acellulaire soluble de *Pasteurella multocida* de type D, de cellules entières inactivées de *P*. *multocida* de type A ou D, de cultures d'*Actinobacillus pleuropneumoniae,* d'*Haemophilus parasuis* et de virus de la pseudo-rage, pour la préparation d'un vaccin pour la vaccination d'un cochon contre *M. hyopneumoniae* et des infections bactériennes secondaires.

16. Utilisation de *M. hyopneumoniae* inactivé, de *Bordetella bronchiseptica* inactivé, de *P. multocida* de type A inactivé, de *P. multocida* de type D inactivé, d'un toxoïde acellulaire de *P*. *multocida*, et d'un extrait d' antigène d' *Erysipelothrix rhusiopathiae*, pour la préparation d'une composition vaccinale pour la vaccination d'un cochon contre *M. hyopneumoniae* et des infections bactériennes secondaires.

17. Utilisation de *M. hyopneumoniae* inactivé avec de l'éthylène imine binaire à une posologie d'au moins 5 x 10⁸ CCU et d'un adjuvant pour la préparation d'un vaccin, et d'un antigène supplémentaire pour la préparation d'un ou plusieurs vaccins pour protéger le porc contre l'infection par *M. hyopneumoniae* et d'autres infections pathogènes, dans laquelle lesdits vaccins doivent être administrés à un animal séquentiellement ou simultanément.

18. Utilisation selon la revendication 17, dans laquelle ledit antigène supplémentaire est choisi dans le groupe constitué d'une bactérine de *Pasteurella multocida* avec un toxoïde fixé aux cellules, d'une bactérine de *Bordetella bronchiseptica*, d'un extrait d'antigène d' *Erysipelothrix rhusiopathiae*, d'un toxoïde acellulaire soluble de *Pasteurella multocida* de type D, de cellules entières inactivées de *P. multocida* de type A ou D, de cultures d'*Actinobacillus pleuropneumoniae,* d'*Haemophilus parasuis* et de virus de la pseudo-rage, pour la préparation d'un vaccin pour la vaccination d'un cochon contre *M. hyopneumoniae* et des infections bactériennes secondaires.

## Revendications (Revendications pour l'(les) Etat(s) contractant(s) suivant(s): ES, GR)

1. Procédé de production d'un vaccin capable d'induire une immunité contre *M. hyopneumoniae* chez un mammifère sans effets secondaires graves, comprenant la combinaison d'une quantité vaccinale d'un composant de vaccin comprenant *M. hyopneumoniae* inactivé avec de l'éthylène imine binaire à une posologie d'au moins 5 x 10⁸ CCU, ledit composant étant capable d'induire une réponse immunologique chez le porc vacciné contre *M. hyopneumoniae*, et d'un adjuvant.

2. Procédé selon la revendication 1, dans lequel le composant comprend le *M. hyopneumoniae* inactivé à un titre compris entre 5 x 10⁸ à 5 x 10¹⁰ unités de changement de couleur (color changing units).

3. Procédé selon la revendication 1, dans lequel ledit adjuvant est choisi dans le groupe constitué de : la lécithine et l'huile minérale, les saponines et l'hydroxyde d'aluminium.

4. Procédé pour la préparation d'un vaccin pour la protection des mammifères contre *M. hyopneumoniae*, qui comprend le prétraitement d'un milieu de culture capable de soutenir *M*. *hyopneumoniae* par une résine d'échange d'anions, l'inoculation dudit milieu avec *M. hyopneumoniae*, l'augmentation de la teneur en oxygène dissout de la dite culture jusqu'à une saturation comprise entre 20 et 40 %, la culture de *M*. *hyopneumoniae* jusqu'à un titre d'au moins 1 x 10⁸ CCU et l'inactivation de la culture par l'ajout d'éthylène imine binaire.

5. Procédé selon la revendication 4, dans lequel ledit titre est compris entre 5 x 10⁸ à. 5 x 10¹⁰ unités de changement de couleur (color changing units).

6. Procédé selon la revendication 4, dans lequel ladite étape de culture comprend la mise en croissance de la culture de *M. hyopneumoniae* à une température comprise entre 30 °C et 40 °C à un pH allant de neutre à légèrement alcalin.

7. Procédé de production d'une composition vaccinale comprenant la combinaison d'un composant de vaccin de *M. hyopneumoniae* inactivé et d'une quantité immunogène d'un ou plusieurs antigènes supplémentaires, dans laquelle lesdits antigènes supplémentaires sont choisis dans le groupe constitué d'une bactérine de *Pasteurella multocida* avec un toxoïde fixé aux cellules, d'une bactérine de *Bordetella bronchiseptica*, d'un extrait d'antigène d'*Erysipelothrix rhusiopathiae*, d'un toxoïde acellulaire soluble de *Pasteurella multocida* de type D, de cellules entières inactivées de *P. multocida* de type A ou D, de cultures d'*Actinobacillus pleuropneumoniae,* d'*Haemophilus parasuis* et de virus de la pseudo-rage.

8. Procédé selon la revendication 7, dans lequel ladite composition vaccinale est constituée de *M. hyopneumoniae* inactivé, de *Bordetella bronchiseptica* inactivé, de *P. multocida* de type A inactivé, de *P. multocida* de type D inactivé, d'un toxoïde acellulaire de *P. multocida* et d'un extrait d'antigène d'*Erysipelothrix rhusiopathiae*.

9. Procédé selon la revendication 7, dans lequel la composition vaccinale est constituée de *M. hyopneumoniae* inactivé et de composants de vaccin d'*Actinobacillus pleuropneumoniae*, d'*Haemophilus parasuis* et de virus de la pseudo-rage

10. Procédé selon la revendication 7, dans lequel la composition vaccinale doit être administrée à un animal séquentiellement ou simultanément.
